# EUROPÄISCHE PATENTANMELDUNG

(11) **EP 1 529 771 A1**
(43) Veröffentlichungstag der Anmeldung: **11.05.2005**
(21) Anmeldenummer: 04025480.7
(22) Anmeldetag: 27.10.2004
(51) Int. Cl.: C07C 47/445, C07C 45/50, C07C 45/82

(54) **Verfahren zur Herstellung von TCD-Monenal**

(30) Priorität: 08.11.2003 DE 10352263
(71) Anmelder: Celanese Chemicals Europe GmbH, 61476 Kronberg (DE)
(72) Erfinder: Lappe, Peter, Dr., 46539 Dinslaken (DE); Springer, Helmut, 46539 Dinslaken (DE); Lukas, Rainer, Dr., 45133 Essen (DE)

(57) **Zusammenfassung**

Die vorliegende Erfindung betrifft ein Verfahren zur selektiven Hydroformylierung von Dicyclopentadien zu 8(9)-Formyl-tricyclo[5.2.1.0^{2.6}]dec-3-en in einem heterogenen Reaktionssystem unter Verwendung einer wässrigen Lösung, die wasserlösliche organische Phosphor(III)-Verbindungen in komplexer Bindung enthaltende Übergangsmetallverbindungen der Gruppe VIII des Periodensystems der Elemente enthält, wobei es sich bei den wasserlöslichen organischen Phosphor(III)-Verbindungen um Alkali- oder Erdalkalisalze sulfonierter Arylphosphine und Aryldiphosphine handelt.

## Beschreibung

Die vorliegende Erfindung betrifft ein Verfahren zur Herstellung von TCD-Monenal {8(9)-Formyl-tricyclo[5.2.1.0^{2.6}]dec-3-en} aus Dicyclopentadien (DCP).

Das durch Dimerisierung von Cyclopentadien leicht zugängliche und auch großtechnisch hergestellte Dicyclopentadien (DCP) lässt sich zu anwendungstechnisch wichtigen Verbindungen umsetzen, denen das Tricyclodecan-Gerüst besondere Eigenschaften verleiht. Die vom DCP-abgeleiteten Verbindungen mit Tricyclodecan-Struktur werden in der Literatur häufig unterschiedlich bezeichnet. In Anlehnung an die aus Chemiker-Zeitung, 98, 1974, Seiten 70 bis 76 bekannte Nomenklatur für DCP-Derivate, wird auch im folgenden die auf dem Tricyclodecan-Gerüst, auch TCD-Gerüst genannt, aufbauende Nomenklatur verwendet.

Insbesondere die Hydroformylierung von DCP liefert interessante TCD-Aldehyde, wie 8(9)-Formyl-tricyclo[5.2.1.0^{2.6}]dec-3-en, auch als TCD-Monenal bezeichnet, oder 3(4),8(9)-Bisformyl-tricyclo[5.2.1.0^{2.6}]decan, auch als TCD-Dialdehyd bezeichnet, die zu wichtigen Zwischenprodukten weiterverarbeitet werden. So ist aus US 4,229,324 bekannt, dass TCD-Monenal mit weiteren Aldehyden zu Folgeprodukten kondensiert wird, die auf dem Riechstoffgebiet Verwendung finden. Nach US 4,087,467 lässt sich TCD-Monenal in Gegenwart von Platinoxid oder Raney Nickel mit Wasserstoff zum gesättigten Monoalkohol reduzieren.

Die Herstellung von Aldehyden durch katalytische Anlagerung von Kohlenmonoxid und Wasserstoff an olefinische Doppelbindungen ist bekannt. Während diese Umsetzung früher nahezu ausschließlich mit Co als Katalysator durchgeführt wurde, arbeiten moderne Verfahren mit metallischem Rhodium oder mit Rhodiumverbindungen als Katalysatoren, die allein oder mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel H[Rh(CO)₄₋ₓLₓ] wiedergeben lassen, wobei L einen Liganden bezeichnet und x gleich 0 oder eine ganze Zahl von 1 bis 3 ist.

Einen Sonderfall stellt die Hydroformylierung von Dienen dar. Während bei der Hydroformylierung konjugierter Diene unter den üblichen Bedingungen der Oxo-Synthese fast ausschließlich Monoaldehyde erhalten werden, lassen sich aus Dicyclopentadien (DCP) mit seinen isolierten Doppelbindungen neben den Mono- auch die Disubstitutionsprodukte gewinnen. Wegen der Gefahr der Retro-Diels-Alder-Reaktion bei den Temperaturen der Oxo-Synthese und der damit verbundenen Freisetzung von Cyclopentadien, das zur Komplexbildung gegenüber Übergangsmetallen befähigt ist und das die Aktivität der eingesetzten Katalysatoren mindern kann, muss die Hydroformylierung unter besonderen Bedingungen ablaufen. Es erwies sich als vorteilhaft, den früher üblichen Co-Katalysator durch Rhodium zu ersetzen, das eine hohe Selektivität der Umsetzung zu Aldehyden erreichen lässt und die Hydroformylierung bei Bedingungen erlaubt, bei denen das Ausmaß der Retro-Diels-Alder-Spaltung geringer ist. Eine zusammenfassende Darstellung über die Hydroformylierung von Dicyclopentadien findet sich in Chemiker-Zeitung 98, 1974, 70-76.

Im Stand der Technik wird auf die thermische Labilität der TCD-Aldehyde hingewiesen, die bei der destillativen Aufarbeitung des rohen Hydroformylierungsgemischs zu hohen Produktverlusten führt. Aufgrund dieser bekannten thermischen Instabilität der TCD-Aldehyde werden diese Aldehyde zumeist nicht rein dargestellt, sondern in ihren Gemischen mit den Nebenprodukten der Oxo-Synthese weiter verarbeitet (Chemikerzeitung, 98 (2), 1974, Seite 72).

In der DE-OS 2 918 107 wird ein Verfahren zur Herstellung von TCD-Monenal beschrieben, in dem Dicyclopentadien unter Katalyse von Rh/TPP und Zusatz von tert. Aminen wie Triethylamin in einem organischen Lösungsmittel umgesetzt wird. Bei der Verwendung von Aminen ist stets mit einer Verunreinigung des TCD-Monenals mit stickstoffhaltigen Komponenten zu rechnen.

Ein Verfahren zur Herstellung von TCD-Monenal durch Hydroformylierung von DCP in Gegenwart von wasserlöslichen, sulfonierten Arylphosphinen ist aus EP-B1-0 186 075 bekannt. Bei diesem, auch als heterogenes Zweiphasenverfahren bekannten Prozeß, verwendet man Katalysatorsysteme, die in Wasser löslich sind. Derartige Katalysatoren sind z.B. in der DE-PS-2 627 354 beschrieben. Die Löslichkeit der Rhodiumkomplexverbindungen wird hierbei durch die Verwendung von sulfonierten Triarylphosphinen als Katalysatorbestandteil erreicht. Die Abtrennung des Katalysators vom Reaktionsprodukt nach Beendigung der Hydroformylierungsreaktion erfolgt bei dieser Verfahrensvariante durch einfache Phasentrennung von wässriger und organischer Phase, d.h. ohne Destillation und damit ohne zusätzliche thermische Verfahrensschritte. In der EP-B1-0 186 075 wird auf einen ungenügenden Teilumsatz beim Einsatz von Rh/TPPTS hingewiesen.

Zur Umsatzerhöhung des nach dem in Gegenwart von Wasser durchgeführten Zweiphasen-Verfahrens schlägt EP-B1-0 186 075 vor, Ammoniumsalze sulfonierter Arylphosphine als Liganden zu verwenden. Nach der Offenbarung von EP-B1-0 186 075 wird durch den Einsatz von Ammoniumsalzen die Löslichkeit von DCP in der wässrigen, katalysatorhaltigen Phase erhöht und die Hydroformylierungsreaktion begünstigt.

Der Einsatz von Ammoniumsalzen in dem Zweiphasen-Hydroformylierungsprozeß stellt jedoch nicht immer die optimale Lösung dar, da Ammoniumsalze im Vergleich zu den Alkali- und Erdalkalisalzen teurer sind und ihr Einsatz aufgrund ihrer grenzflächenaktiven Wirkung zu Schwierigkeiten bei der Phasentrennung zwischen der organischen aldehydhaltigen und der wässrigen katalysatorhaltigen Phase führen kann. Eine unscharfe Phasentrennung in dem Zweiphasen-Hydroformylierungsprozeß ist mit Nachteilen behaftet, da es zu Edelmetallverlusten über den Austrag in die organische Phase kommen kann und die wässrige katalysatorhaltige Phase übermäßig mit organischen Produkten belastet wird, so dass eine spätere Aufarbeitung der gebrauchten wässrigen Altkatalysatorlösungen aufgrund des hohen Anteils organischer Verbindungen verkompliziert wird. Ferner können N-haltige Katalysatorkomponenten in der organischen Phase zu Problemen bei der Weiterverarbeitung führen, insbesondere beim Einsatz auf dem Riechstoffsektor.

Es besteht daher ein Bedarf nach einem kostengünstigen und einfachen Verfahren zur Herstellung von TCD-Monenal, das die geschilderten Nachteile überwindet.

Die Erfindung besteht daher in einem Verfahren zur Herstellung von 8(9)-Formyl-tricyclo[5.2.1.0]^{2.6}]dec-3-en durch Hydroformylierung von Dicyclopentadien in einem heterogenen Reaktionssystem unter Verwendung einer wässrigen Lösung, wasserlösliche organische Phosphor(III)verbindungen in komplexer Bindung enthaltender Übergangsmetallverbindungen der Gruppe VIII des Periodensystems der Elemente bei Temperaturen von 70 bis 150°C und Drücken von 0,5 bis 10 MPa mit Synthesegas. Es ist dadurch gekennzeichnet, dass man als wasserlösliche organische Phosphor(III)-Verbindungen sulfonierte Triarylphosphine der allgemeinen Formel (I) 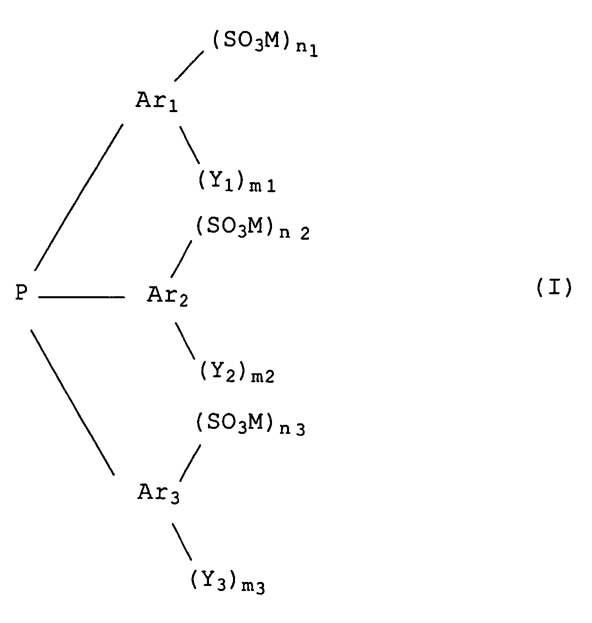 in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist; dass man als wasserlösliche organische Phosphor(III)-Verbindungen sulfonierte Diphosphine der allgemeinen Formel II

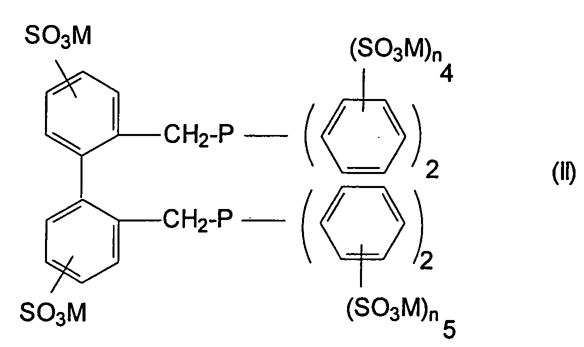

in der jedes n₄ und n₅ unabhängig voneinander für 0 oder 1 steht, wobei die Verbindung der Formel (II) bis sechs -SO₃M-Gruppe enthält, und in der M für ein einwertiges Metall oder das Äquivalent eines mehrwertigen Metalls steht; oder dass man als wasserlösliche organische Phospor(III)-Verbindungen sulfonierte Diphosphine der allgemeinen Formel (III) 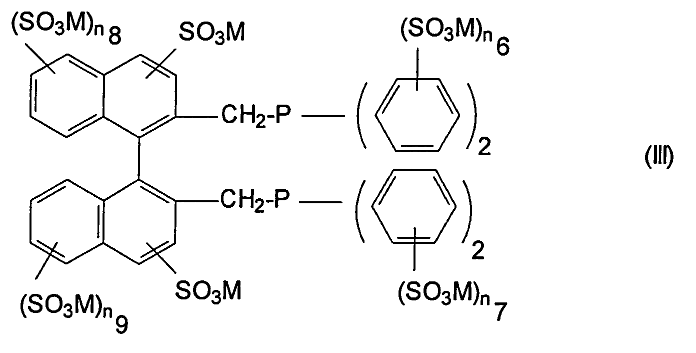 in der jedes n₆, n₇, n₈ und n₉ unabhängig voneinander für 0 oder 1 steht, wobei die Verbindung der Formel (III) vier bis acht -SO₃M-Gruppen enthält, und in der M für ein einwertiges Metall oder das Äquivalent eines mehrwertigen Metalls steht, verwendet.

Überraschenderweise und im Gegensatz zu der Lehre aus EP-B1-0 186 075 zeigt sich, dass bei der Herstellung von TCD-Monenal auch dann hohe Ausbeuten erzielt werden können, wenn anstelle von Ammoniumsalzen sulfonierter Arylphosphine, Alkali- oder Erdalkalisalze sulfonierter Arylphosphine verwendet werden.

Das den Hydroformlyierungsreaktor verlassende Reaktionsgemisch trennt sich in dem nachgeschalteten Phasentrenner innerhalb kurzer Zeit in zwei klare Phasen, in die organische, aldehydhaltige Phase und in die wässrige, katalysatorhaltige Phase. Durch die klare Phasentrennung wird der Rhodiumaustrag in die organische Phase und der Eintrag an Organika in die wässrige Phase minimiert.

Das nach dem erfindungsgemäßen Verfahren hergestellte TCD-Monenal kann als Rohprodukt ohne weitere Reinigungsschritte weiterverarbeitet werden. Dies ist insofern überraschend, weil die das Wertprodukt enthaltende organische Phase homogen gelöste und analytisch nachweisbare Mengen an Phosphor- und Schwefel-Spalt- und Abbauprodukten enthält, die als Katalystorgifte für viele Reaktionen bekannt sind.

Ebenfalls ist eine destillative Reinigung des nach dem erfindungsgemäßen Verfahren hergestellten rohen TCD-Monenals in hohen Ausbeuten möglich. Im Gegensatz zu der bisher im Stand der Technik geäußerten Auffassung, dass die Destillation von TCD-Aldehyden aufgrund der thermischen Labilität zu Produktverlusten führt (Chemiker-Zeitung, 98 (2), 1974, Seite 72), lässt sich nach dem erfindungsgemäßen Verfahren hergestelltes rohes TCD-Monenal unter nur sehr geringen Verlusten destillativ in hoher Reinheit gewinnen.

Die Hydroformylierungsreaktion von DCP führt man als heterogene Reaktion im einem Zweiphasensystem durch, eine Umsetzung, die z.B. in der DE-B-26 27 354 beschrieben ist. Dieser Prozess ist charakterisiert durch das Vorliegen einer organischen Phase, die das olefinische Ausgangsmaterial und das Reaktionsprodukt enthält, und einer wässrigen Phase, in der der Katalysator gelöst ist. Als Katalysatoren werden wasserlösliche Rhodium-Komplexverbindungen eingesetzt, die wasserlösliche organischen Phosphor(III)-Verbindungen als Liganden enthalten. Als wasserlösliche organische Phosphor(III)-Verbindungen verwendet man sulfonierte Triarylphosphine der allgemeinen Formel (I)

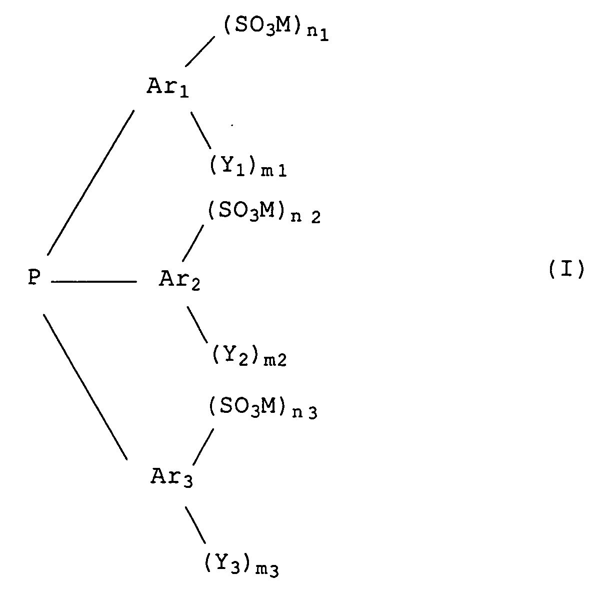

in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

Zu den Triarylphosphinen der allgemeinen Formel (I) zählen bevorzugt solche Triarylphosphine, bei denen die Gruppen Ar¹, Ar², Ar³ Phenylgruppen sind; Y₁, Y₂ und Y₃ für die Methyl-, die Ethylgruppe, für die Methoxy-, Ethoxygruppe und/oder für ein Chloratom stehen; und die kationischen Reste M anorganische Kationen von Natrium, Kalium, Calcium und Barium sind. Insbesondere geeignet sind solche Triarylphosphine, bei denen Ar¹, Ar², Ar³ jeweils eine Phenylgruppe bedeuten, m₁, m₂, m₃ gleich 0 sind, n₁, n₂ und n₃ gleich 0 oder 1 sind und n₁+n₂+n₃ zusammen 1 bis 3 ausmachen, und bei denen die Sulfonat-Gruppen in meta-Stellung stehen.

Ein für die Durchführung des erfindungsgemäßen Hydroformylierungsverfahrens geeignetes Gemisch an (Sulfophenyl)-diphenylphosphin, Di-(sulfophenyl)phenylphosphin und Tri(sulfophenylphosphin) fällt bei der Sulfonierung von Triphenylphosphin an, wie z.B. aus DE-OS 26 27 354 bekannt. Im Stand der Technik wird (Sulfophenyl)diphenylphosphin als TPPMS, Di-(sulfophenyl)phenylphosphin als TPPDS und Tri(sulfophenyl)phosphin als TPPTS abgekürzt.

Als wasserlösliche organische Phosphor(III)-Verbindungen eignen sich ebenfalls sulfonierte Diphosphine der allgemeinen Formeln (II) oder (III)

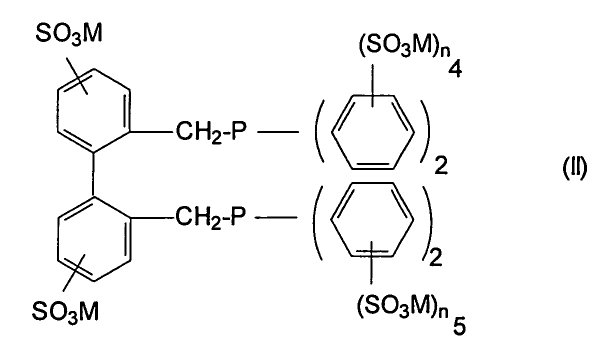

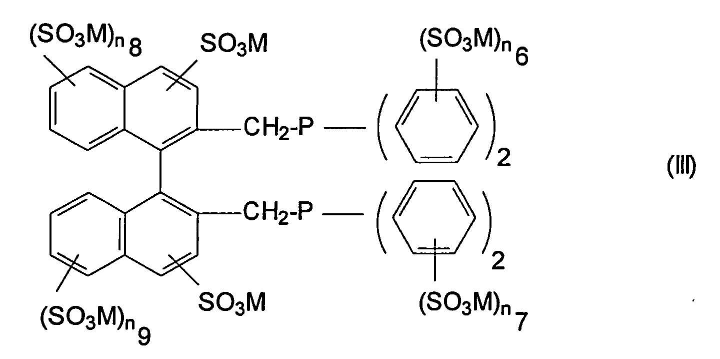

Diese Diphosphine der allgemeinen Formeln (II) und (III) sind aus WO98/30526 bekannt.

In (II) steht ein jedes n₄ und n₅ unabhängig voneinander für 0 oder 1, wobei die Verbindung der Formel (II) bis sechs -SO₃M-Grup enthält.

In (III) steht ein jedes n₆, n₇, n₈ und n₉ unabhängig voneinander für 0 oder 1, wobei die Verbindung der Formel (III) vier bis acht -SO₃M-Gruppe enthält.

Aufgrund der Herstellung durch Sulfonierung der entsprechenden Diphosphine der Formeln (IIa) und (IIIa), die keine -SO₃M-Gruppen enthalten, 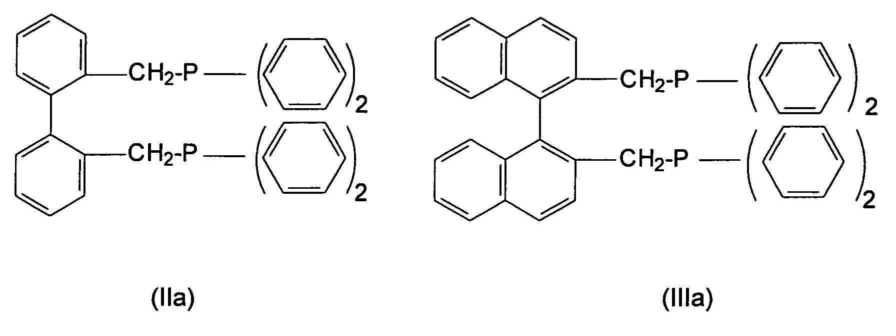 erhält man üblicherweise Gemische von Verbindungen (II) und (III) mit unterschiedlicher Anzahl von -SO₃M-Gruppen. So enthält eine Verbindung der Formeln (II) oder (III), die beispielsweise drei -SO₃M-Gruppen enthält, auch Verbindungen mit lediglich zwei -SO₃M-Gruppen aber auch Verbindungen mit vier oder fünf -SO₃M-Gruppen. Eine Verbindung der Formeln (II) oder (III) mit beispielsweise fünf -SO₃M-Gruppen enthält üblicherweise auch Verbindungen mit lediglich drei oder vier -SO₃M-Gruppen aber auch Verbindungen mit sechs oder sieben -SO₃M-Gruppen.

Verbindungen der Formel (II) besitzen maximal sechs -SO₃M-Gruppen, während Verbindungen der Formel (III) maximal acht -SO₃M-Gruppen aufweisen.

Aus diesem Grund gelangen in der Regel Mischungen von Verbindungen der Formeln (II) und (III) mit unterschiedlicher Anzahl von -SO₃M-Gruppen zum Einsatz.

In den Formeln (II) und (III) steht M für ein einwertiges Metall oder das Äquivalent eines mehrwertigen Metalls, insbesondere für Lithium, Natrium, Kalium, Magnesium, Calcium oder Barium.

Besonders vorteilhaft ist der Einsatz wasserlöslicher Komplexverbindungen des Rhodiums, obwohl die Verwendung anderer katalytisch aktiver Übergangsmetallverbindungen der Gruppe VIII des Periodensystems der Elemente nicht ausgeschlossen wird. So kann man auch wasserlösliche Komplexverbindungen von Kobalt, Iridium, Nickel, Palladium, Platin, Eisen oder Ruthenium verwenden, und besonders wasserlösliche Komplexverbindungen des Kobalts, Iridiums und Platins haben sich als wirksame Hydroformylierungskatalysatoren erwiesen.

Die Bedingungen, unter denen die Umsetzung abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung der Einsatzstoffe bei Temperaturen von 70 bis 150°C durch. Bevorzugt hält man Temperaturen von 100 bis 150°C und insbesondere von 110 bis 140°C ein. Der Gesamtdruck erstreckt sich über einen Bereich von 0,5 bis 10 MPa, vorzugsweise 1 bis 6 MPa und insbesondere 1,5 bis 5 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Die Rhodium-Konzentration beträgt 20 bis 1000 Gew.-ppm, vorzugsweise 50 bis 800 Gew.-ppm und insbesondere 100 bis 600 Gew.-ppm, jeweils bezogen auf die wässrige Katalysatorlösung. Obgleich es möglich ist, als Katalysator die stöchiometrisch zusammengesetzte Rhodium-Phosphor-Komplexverbindung einzusetzen, arbeitet man üblicherweise in Gegenwart von überschüssigem Phosphor-Liganden, d.h. Ligand, der mit Rhodium keine komplexe Bindung eingegangen ist. Je mol Rhodium wendet man bevorzugt 10 bis 300 mol Phosphor in Form einer wasserlöslichen organischen Phosphor(III)-Verbindung an. Besonders bewährt haben sich molare Verhältnisse von Rhodium zu Phosphor im Bereich von 1:50 bis 1:150. Der Rhodium-Phosphor-Komplexkatalysator braucht nicht einheitlich zusammengesetzt sein, sondern kann z.B. aus einem Gemisch von Rhodium-Komplexverbindungen bestehen, die sich durch die Art der Phosphor-Liganden unterscheiden. Ebenso kann der in der wässrigen Katalysatorlösung enthaltene freie Phosphor-Ligand aus einem Gemisch unterschiedlicher wasserlöslicher organischer Phosphorverbindungen zusammengesetzt sein.

Wird ein anderes Übergangsmetall der Guppe VIII des Periodensystems der Elemente als katalytisch aktives Metall eingesetzt, so bewegt sich die Konzentration an Übergangsmetall sowie das molare Verhältnis von Übergangsmetall zu Phosphor in den Bereichen, die man auch bei Rhodium wählt. Die jeweiligen optimalen Werte lassen sich in Abhängigkeit von dem jeweils eingesetzten Übergangsmetall durch einfache Routineversuche ermitteln.

Man bildet den Katalysator üblicherweise aus den Komponenten Übergangsmetall oder Übergangsmetallverbindung, organische Phosphorverbindung und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Dicylopentadien kann als solches oder in Lösung der Hydroformylierung zugeführt werden. Geeignete Lösungsmittel sind wasserunlösliche Ketone, Dialkylether, aliphatische Nitrile, aromatische Kohlenwasserstoffe wie Benzol oder Toluol und gesättigte cycloaliphatische Kohlenwasserstoffe wie Cyclopentan oder Cylcohexan oder gesättigte aliphatische Kohlenwasserstoffe.

Auch hinsichtlich der verfahrenstechnischen und apparativen Ausgestaltung des neuen Verfahrens kann man sich innerhalb weiter Grenzen bewegen. Eine bewährte Ausführungsform der heterogenen Hydroformylierung unter Verwendung einer wässrigen Katalysatorphase ist in der EP-B1-0 103 810 beschrieben. Der Reaktionsaustrag der Hydroformylierungsstufe wird in einem Phasentrenner in die organische Produktphase und in die wässrige Katalysatorlösung aufgetrennt. Es hat sich als zweckmäßig erwiesen, die Katalysatorlösung im Kreis zu führen. Die rohe organische Produktphase kann anschließend ohne weitere Reinigungsschritte für Nachfolgereaktionen eingesetzt werden oder vor der Weiterverarbeitung destillativ aufgearbeitet werden.

Zur Herstellung des Hydroformylierungskatalysators gelangt das Übergangsmetall der Gruppe VIII des Periodensystems der Elemente, insbesondere Rhodium, entweder in metallischer Form oder als Verbindung zum Einsatz. In metallischer Form verwendet man das Übergangsmetall entweder als feinverteilte Partikel oder in dünner Schicht auf einem Träger, wie Aktivkohle, Calciumcarbonat, Aluminiumsilikat, Tonerde niedergeschlagen. Als Übergangsmetallverbindungen eignen sich Salze aliphatischer Mono- und Polycarbonsäuren, wie Übergangsmetall-2-Ethylhexanoate, -Acetate, -Oxalate, -Propionate oder -Malonate. Weiterhin können Salze anorganischer Wasserstoff- und Sauerstoffsäuren, wie Nitrate oder Sulfate, die verschiedenen Übergangsmetalloxide oder auch Übergangsmetallcarbonylverbindungen, wie Rh₃(CO)₁₂, Rh₆(CO)₁₆, Co₂(CO)₈, Co₄(CO)₁₆, Fe(CO)₅, Fe₂(CO)₉, Ir₂(CO)₈, Ir₄(CO)₁₂ oder Übergangsmetall-Komplexverbindungen, z.B. Cyclopentadienylrhodiumverbindungen, Rhodiumacetylacetonat, Cyclopentadienylkobalt-Cyclooctadien-1,5, Fe(CO)₃-Cyclooctadien-1,5, [RhCl(Cyclooctadien-1,5]₂ oder PtCl₂(Cyclooctadien-1,5) eingesetzt werden. Übergangsmetallhalogenverbindungen kommen wegen ihres korrosiven Verhaltens der Halogenidionen weniger in Betracht.

Bevorzugt werden Übergangsmetalloxide und insbesondere Übergangsmetallacetate und -2-ethylhexanoate. Als besonders geeignet hat sich Rhodiumoxid, Rhodiumacetat, Rhodium-2-ethylhexanoat, Kobaltoxid, Kobaltacetat und Kobalt-2-ethylhexanoat erwiesen.

Die Hydroformylierungsreaktion kann sowohl absatzweise als auch kontinuierlich durchgeführt werden.

Wird das Reaktionsprodukt der Hydroformylierungsreaktion destilliert, arbeitet man im allgemeinen bei einer Kopftemperatur in einem Bereich von 140 bis 142°C und bei einem Druck in einem Bereich von 50 hPa. TCD-Monenal wird in einer Reinheit von >99 % erhalten. Die Destillationsverluste betragen weniger als 3 %.

Das erfindungsgemäße Verfahren gestattet einen einfachen und kostengünstigen Zugang zu TCD-Monenal in hoher Ausbeute und in hoher Reinheit. Das nach dem erfindungsgemäßen Verfahren hergestellte TCD-Monenal lässt sich in ausgezeichneter Weise für unterschiedliche Anwendungen einsetzen.

Im folgenden wird das erfindungsgemäße Verfahren anhand einiger Beispiele näher erläutert, es ist jedoch nicht auf die beschriebenen Ausführungsformen beschränkt.

### Beispiele

Die bei der analytischen Charakterisierung der Reaktionsprodukte verwendeten Abkürzungen haben folgende Bedeutung:

| | |
|---|---|
| DCP | Dicyclopentadien |
| TCD-Monenal | 8(9)-Formyl-tricyclo[5.2.1.0^{2.6}]dec-3-en |
| TCD-Dial | 3(4),8(9)-Bisformyl-tricyclo[5.2.10^{2.6}]decan |
| Tri-CP | Tricyclopentadien. |
| | |
| TPPTS bedeutet | Natrium-Triphenylphosphintrisulfonat |

### Herstellung von TCD-Monenal

### Beispiel 1

In einem 5 1-Autoklaven werden 2.119 g TPPTS-Lösung mit einem P(III)-Gehalt von 472 mmol/kg vorgelegt und mit 160,2 g Rh-Lösung (Rh-Gehalt: 6.423 mg/kg) versetzt. Danach wird eine Mischung aus 661,1 g Dicyclopentadien (technisch, DCP-Gehalt: 93,72 Gew.-%) und 283,0 g Toluol zugegeben. Das Reaktionsgemisch wird auf 135°C erwärmt und bei einem Synthesegasdruck von 2,5 MPa und einer Reaktionszeit von 6 Stunden umgesetzt.

Nach Reaktionsende wird abgekühlt und die obere, organische Phase von der wässrigen Katalysatorphase durch Phasentrennung abgetrennt. Die verbleibende Katalysatorphase wird erneut mit einem Gemisch aus Dicyclopentadien und Toluol versetzt und erneut umgesetzt. Dieser Vorgang wird insgesamt achtmal wiederholt.

Die organischen Phasen werden ausgewogen und gaschromatographisch untersucht.

| GC-Analyse (in FI.-%, Flächenprozent) | | | | | |
|---|---|---|---|---|---|
| | 1. Einsatz | 3. Einsatz | 5. Einsatz | 7. Einsatz | 9. Einsatz |
| Vorlaufkomponenten | 0,32 | 0,31 | 0,34 | 0,37 | 0,37 |
| Toluol | 39,22 | 27,03 | 29,45 | 29,47 | 29,96 |
| DCP | 3,66 | 4,13 | 4,55 | 4,60 | 5,24 |
| TCD-Monenal | 52,85 | 63,80 | 61,20 | 61,16 | 59,94 |
| TCD-Dial | 0,56 | 0,95 | 0,80 | 0,83 | 0,82 |
| Tri-CP | 0,39 | 0,45 | 0,46 | 0,41 | 0,42 |
| Sonstige | 3,00 | 3,33 | 3,20 | 3,16 | 3,25 |
| Org. Phase (g) | 1.158 | 1.091 | 1.073 | 1.070 | 1.086 |
| Ausbeute TCD-Monenal (%) | | 92,9 | 90,1 | 89,7 | 90,4 |

Der erhöhte Toluolwert im 1. Einsatz ist durch das in der Rh-Lösung enthaltene Toluol bedingt und wird nach dem 1. Einsatz mit der organischen Wertproduktphase ausgetragen und wird bei der Betrachtung der Ausbeuten nicht berücksichtigt. Die Dickölgehalte (bestimmt durch eine Flashdestillation an einer Claisenbrücke) liegen bei allen Versuchen unter 2 Gew-%.

Die Ausbeuten an TCD-Monenal liegen in dieser Versuchsreihe (Versuche 3 - 9) bei 90 - 93 %, der Durchschnittswert beträgt 90,8 %.

### Beispiel 2

Die aus Beispiel 1 verbliebene Rh/TPPTS-Katalysatorlösung wird gemäß der Arbeitsweise aus Beispiel 1 mit 661,1 g Dicyclopentadien (technisch, DCP-Gehalt: 93,72 Gew.-%) versetzt und unter gleichen Reaktionsbedingungen (135°C, 2,5 MPa, 6 Stunden) umgesetzt.

Die organischen Phasen werden ausgewogen und gaschromatographisch untersucht.

| GC-Analyse (in FI.-%) | | | | |
|---|---|---|---|---|
| | 10. Einsatz | 11. Einsatz | 13. Einsatz | 15. Einsatz |
| Vorlaufkomponenten | 0,26 | 0,32 | 0,33 | 0,35 |
| Toluol | 0,74 | 0,12 | 0,01 | <0.01 |
| DCP | 1,37 | 2,71 | 2,84 | 3,47 |
| TCD-Monenal | 90,18 | 89,37 | 89,34 | 88,85 |
| TCD-Dial | 1,74 | 1,66 | 1,49 | 1,49 |
| Tri-CP | 0,50 | 0,53 | 0,62 | 0,55 |
| Sonstige | 5,21 | 5,29 | 5,37 | 5,28 |
| | | | | |
| Org. Phase (g) | 786 | 807 | 802 | 808 |
| Ausbeute TCD-Monenal (%) | 94,0 | 95,0 | 94,2 | 94,4 |

Die Dickölgehalte (bestimmt durch eine Flashdestillation an einer Claisenbrücke) liegen bei allen Versuchen unter 2 Gew.-%.

Die Ausbeuten an TCD-Monenal liegen in dieser Versuchsreihe (Versuche 10 - 15) bei 94 - 95 %, der Durchschnittswert beträgt 94,4 %.

### Beispiel 3

Die destillative Reingewinnung von TCD-Monenal erfolgt an einer Kolonne mit 4,5 theoretischen Böden, hierfür wird folgendes typisches Hydroformylierungsprodukt (800,9 g) eingesetzt:

| GC-Analyse (in FI.-%) | |
|---|---|
| Vorlaufkomponenten | 0,61 |
| Toluol | 29,81 |
| DCP | 3,29 |
| TCD-Monenal | 62,06 |
| TCD-Dial | 0,67 |
| Tri-CP | 0,29 |
| Sonstige | 3,27 |

Nach Abtrennung von Vorlaufkomponenten, Lösemittel (Toluol) und Zwischenlaufkomponenten (in Summe 302,5 g) bei einem Rücklaufverhältnis (RLV) von 2:1 bzw. 5:1, einer max. Kopftemperatur von 140°C und einem Druck von 50 hPa, wird eine Reinfraktion (482,9 g) in einem Siedebereich von 141 - 142°C (Kopftemperatur) bei 50 hPa und einem RLV von 0,5 : 1 mit folgender Zusammensetzung isoliert.

| GC-Analyse (in FI-%) | |
|---|---|
| Toluol | 0,02 |
| DCP | 0,03 |
| TCD-Monenal | 99,02 |
| TCD-Dial | 0,04 |
| Tri-CP | 0,20 |
| Sonstige | 0,69 |

Dies entspricht einer destillativen Ausbeute von 96,2 %. Die Rückstandsmenge beträgt 15,5 g (1,9 % vom Einsatz).

Wie das Beispiel 3 zeigt, lässt sich TCD-Moneal mit einer hervorragenden Ausbringung destillativ reinigen.

### Beispiel 4

Gemäß Beispiel 1 werden 2.119 g TPPTS-Lösung (P(III)-Gehalt: 472 mmol/kg) und 160,2 g Rh-2-ethylhexanoat-Lösung (Rh-Gehalt: 6.423 mg/kg) vorgelegt. Zu dieser Lösung werden 661,1 g Dicyclopentadien (DCP-Gehalt: 93,72 Gew.-%) und 283 g Toluol gegeben und bei Temperaturen von 120 - 135°C und einer Reaktionszeit von 6 Stunden zur Reaktion gebracht.

Die organischen Phasen werden ausgewogen und GC-analytisch untersucht.

| GC-Analyse (in FI.-%) | | | | |
|---|---|---|---|---|
| Temperatur (°C) | 135 | 130 | 125 | 120 |
| Vorlaufkomponenten | 0,67 | 0,65 | 0,61 | 0,56 |
| Toluol | 32,80 | 32,20 | 31,10 | 31,50 |
| DCP | 3,99 | 8,09 | 14,01 | 21,23 |
| TCD-Monenal | 58,71 | 56,03 | 51,91 | 44,52 |
| TCD-Dial | 0,94 | 0,75 | 0,49 | 0,44 |
| Tri-CP | 0,55 | 0,40 | 0,36 | 0,31 |
| Sonstige | 2,34 | 1,88 | 1,52 | 1,44 |
| | | | | |
| Org. Phase (g) | 1.072 | 1.068 | 1.058 | 1.062 |
| Selektivität (%) | 96,4 | 96,8 | 96,4 | 96,5 |

### Beispiel 5

Gemäß Beispiel 1 werden 2.119 g TPPTS-Lösung (P(III)-Gehalt: 472 mmol/kg) und 160,2 g Rh-2-ethylhexanoat-Lösung (Rh-Gehalt: 6.423 mg/kg) vorgelegt. Zu dieser Lösung werden 661,1 g Dicyclopentadien (DCP-Gehalt: 93,72 Gew.-%) und 283 g Toluol gegeben und bei einer Temperaturen von 125°C und einem Druck von 2,5 MPa zur Reaktion gebracht. Über einen Zeitraum von 6 - 15 Stunden werden Proben entnommen und gaschromatographisch untersucht.

| GC-Analyse (in FI.-%) | | | | | |
|---|---|---|---|---|---|
| Reaktionszeit (Std.) | 6 | 8 | 10 | 12 | 15 |
| Vorlaufkomponenten | 0,61 | 0,58 | 0,62 | 0,56 | 0,55 |
| Toluol | 31,10 | 29,60 | 30,15 | 29,60 | 29,25 |
| DCP | 14,01 | 9,62 | 5,82 | 3,50 | 1,73 |
| TCD-Monenal | 51,91 | 57,45 | 60,62 | 63,12 | 64,92 |
| TCD-Dial | 0,49 | 0,53 | 0,61 | 0,83 | 1,09 |
| Tri-CP | 0,36 | 0,40 | 0,40 | 0,42 | 0,39 |
| Sonstige | 1,52 | 1,82 | 1,78 | 1,97 | 2,07 |

### Beispiel 6

Gemäß Beispiel 1 werden 2.119 g TPPTS-Lösung (P(III)-Gehalt: 472 mmol/kg) und 160,2 g Rh-2-ethylhexanoat-Lösung (Rh-Gehalt: 6.423 mg/kg) vorgelegt. Zu dieser Lösung werden 661,1 g Dicyclopentadien (DCP-Gehalt: 93,72 Gew.-%) und 283 g Toluol gegeben und bei einer Temperaturen von 130°C und einem Druck von 2,5 MPa zur Reaktion gebracht. Über einen Zeitraum von 4 - 10 Stunden werden Proben entnommen und gaschromatographisch untersucht.

| GC-Analyse (in FI.-%) | | | | |
|---|---|---|---|---|
| Reaktionszeit (Std.) | 4 | 6 | 8 | 10 |
| Vorlaufkomponenten | 0,80 | 0,65 | 0,75 | 0,62 |
| Toluol | 30,40 | 31,20 | 30,50 | 30,70 |
| DCP | 16,36 | 8,09 | 4,31 | 2,36 |
| TCD-Monenal | 49,76 | 57,03 | 60,99 | 62,89 |
| TCD-Dial | 0,59 | 0,75 | 0,94 | 1,00 |
| Tri-CP | 0,28 | 0,40 | 0,38 | 0,42 |
| Sonstige | 1,81 | 1,88 | 2,13 | 2,01 |

### Beispiel 7

Gemäß Beispiel 1 werden 874 g TPPTS-Lösung (P(III)-Gehalt: 472 mmol/kg), 66,0 g Rh-2-ethylhexanoat-Lösung (Rh-Gehalt: 6.423 mg/kg) und 1.245 g Wasser vorgelegt. Zu dieser Lösung werden 661,1 g Dicyclopentadien (DCP-Gehalt: 93,72 Gew.-%) und 283 g Toluol gegeben und bei einer Temperaturen von 125°C und einem Druck von 2,5 MPa zur Reaktion gebracht. Über einen Zeitraum von 6 - 16 Stunden werden Proben entnommen und gaschromatographisch untersucht.

| GC-Analyse (in FI.-%) | | | | | |
|---|---|---|---|---|---|
| Reaktionszeit (Std.) | 6 | 8 | 10 | 12 | 14 |
| Vorlaufkomponenten | 0,42 | 0,35 | 0,21 | 0,07 | 0,14 |
| Toluol | 30,61 | 29,95 | 29,76 | 30,05 | 29,40 |
| DCP | 14,50 | 8,34 | 4,85 | 2,87 | 1,34 |
| TCD-Monenal | 52,26 | 58,84 | 62,43 | 64,07 | 65,87 |
| TCD-Dial | 0,42 | 0,56 | 0,70 | 0,77 | 0,99 |
| Tri-CP | 0,28 | 0,28 | 0,28 | 0,35 | 0,28 |
| Sonstige | 1,51 | 1,68 | 1,76 | 1,82 | 1,98 |

### Beispiel 8

Gemäß Beispiel 1 werden 874,0 g TPPTS-Lösung (P(III)-Gehalt: 472 mmol/kg), 64,3 g Rh-2-ethylhexanoat-Lösung (Rh-Gehalt: 6.603 mg/kg) und 1.245,0 g Wasser vorgelegt. Zu dieser Lösung werden 661,1 g Dicyclopentadien (DCP-Gehalt: 93,72 Gew.-%) und 283 g Toluol gegeben und bei einer Temperaturen von 125°C und einem Druck von 2,5 MPa zur Reaktion gebracht. Über einen Zeitraum von 8 - 15 Stunden werden Proben entnommen und gaschromatographisch untersucht.

| GC-Analyse (in FI.-%) | | |
|---|---|---|
| Reaktionszeit (Std.) | 8 | 15 |
| Vorlaufkomponenten | 0,92 | 0,63 |
| Toluol | 29,55 | 29,83 |
| DCP | 8,38 | 1,89 |
| TCD-Monenal | 58,90 | 63,80 |
| TCD-Dial | 0,49 | 0,42 |
| Tri-CP | 0,49 | 1,12 |
| Sonstige | 1,27 | 2,31 |

### Beispiel 9

Gemäß Beispiel 1 werden 654,0 g TPPTS-Lösung (P(III)-Gehalt: 472 mmol/kg), 48,1 g Rh-2-ethylhexanoat-Lösung (Rh-Gehalt: 6.603 mg/kg) und 1.465,0 g Wasser vorgelegt. Zu dieser Lösung werden 661,1 g Dicyclopentadien (DCP-Gehalt: 93,72 Gew.-%) und 283 g Toluol gegeben und bei einer Temperaturen von 125°C und einem Druck von 2,5 MPa zur Reaktion gebracht. Nach einer Reaktionszeit von 8 Stunden wird das Reaktionsgemisch gaschromatographisch untersucht.

| GC-Analyse (in FI.-%) | |
|---|---|
| Vorlaufkomponenten | 0,56 |
| Toluol | 30,30 |
| DCP | 1,53 |
| TCD-Monenal | 64,19 |
| TCD-Dial | 1,05 |
| Tri-CP | 0,35 |
| Sonstige | 2,02 |

### Beispiel 10

Gemäß Beispiel 1 werden 436,0 g TPPTS-Lösung (P(III)-Gehalt: 472 mmol/kg), 32,1 g Rh-2-ethylhexanoat-Lösung (Rh-Gehalt: 6.603 mg/kg) und 1.683,0 g Wasser vorgelegt. Zu dieser Lösung werden 661,1 g Dicyclopentadien (DCP-Gehalt: 93,72 Gew.-%) und 283 g Toluol gegeben und bei einer Temperaturen von 125°C und einem Druck von 2,5 MPa zur Reaktion gebracht. Nach einer Reaktionszeit von 16 Stunden wird das Reaktionsgemisch gaschromatographisch untersucht.

| GC-Analyse (in FI.-%) | |
|---|---|
| Vorlaufkomponenten | 0,78 |
| Toluol | 29,50 |
| DCP | 3,45 |
| TCD-Monenal | 62,60 |
| TCD-Dial | 0,85 |
| Tri-CP | 0,56 |
| Sonstige | 2,26 |

Wie die Beispiele zeigen, lässt sich Dicyclopentadien in Gegenwart einer wässrigen Natrium-TPPTS-Lösung in hohen Ausbeuten selektiv in TCD-Monenal überführen. Bei der Phasentrennung zwischen der organischen Wertproduktphase und der wässrigen Katalysatorphase treten keine Phasentrennprobleme auf. Bei der destillativen Reinigung des Rohproduktes fallen Rückstände in nur sehr geringer Menge an.

## Patentansprüche

1. Verfahren zur Herstellung von 8(9)-Formyl-tricyclo[5.2.1.0^{2.6}]dec-3-en durch Hydroformylierung von Dicyclopentadien in einem heterogenen Reaktionssystem unter Verwendung einer wässrigen Lösung, wasserlösliche organische Phosphor(III)verbindungen in komplexer Bindung enthaltender Übergangsmetallverbindungen der Gruppe VIII des Periodensystems der Elemente bei Temperaturen von 70 bis 150°C und Drücken von 0,5 bis 10 MPa mit Synthesegas, **dadurch gekennzeichnet, dass** man als wasserlösliche organische Phosphor(III)-Verbindungen sulfonierte Triarylphosphine der allgemeinen Formel (I) 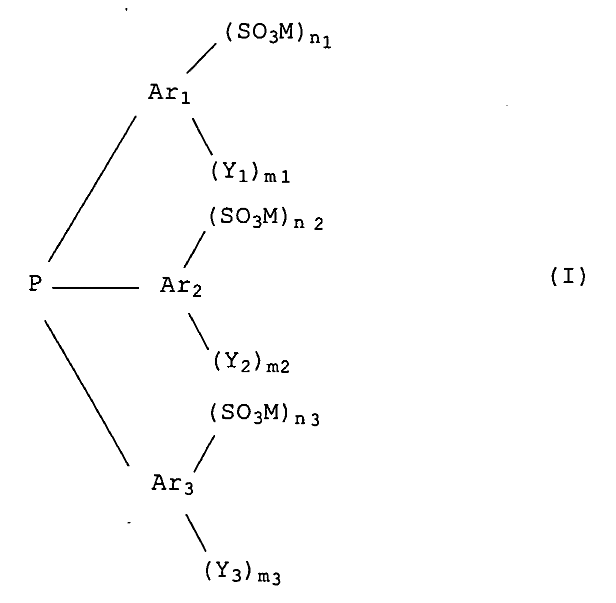 in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist; dass man als wasserlösliche organische Phosphor(lll)-Verbindungen sulfonierte Diphosphine der allgemeinen Formel 11 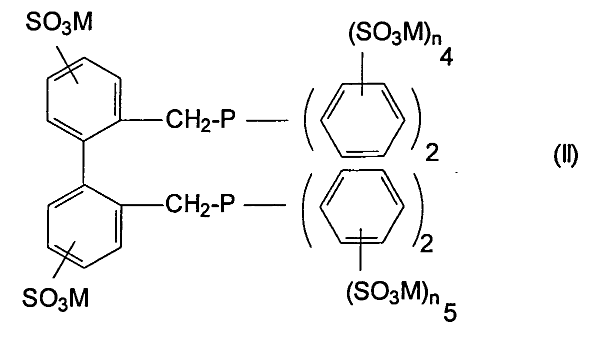 in der jedes n₄ und n₅ unabhängig voneinander für 0 oder 1 steht, wobei die Verbindung der Formel (II) bis sechs -SO₃M-Grup enthält, und in der M für ein einwertiges Metall oder das Äquivalent eines mehrwertigen Metalls steht; oder dass man als wasserlösliche organische Phospor(III)-Verbindungen sulfonierte Diphosphine der allgemeinen Formel (III) 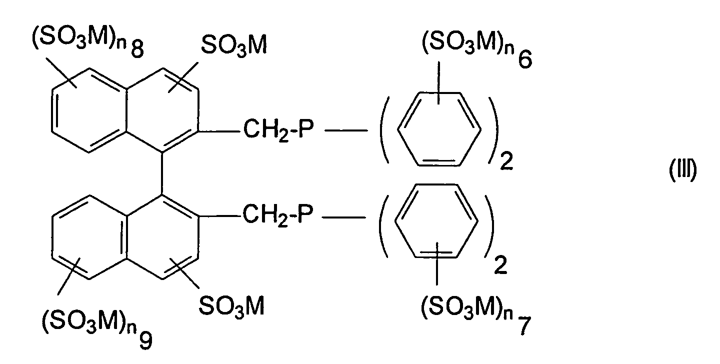 in der jedes n₆, n₇, n₈ und n₉ unabhängig voneinander für 0 oder 1 steht, wobei die Verbindung der Formel (III) vier bis acht -SO₃M-Gruppe enthält, und in der M für ein einwertiges Metall oder das Äquivalent eines mehrwertigen Metalls steht, verwendet.

2. Verfahren gemäß Anspruch 1, **dadurch gekennzeichnet, dass** das erhaltene 8(9)-Formyl-tricyclo[5.2.1.0^{2.6}]dec-3-en destilliert wird.

3. Verfahren gemäß Anspruch 1, **dadurch gekennzeichnet, dass** in der allgemeinen Formel (I) Ar₁, Ar₂, Ar₃ jeweils eine Phenylgruppe bedeuten, m₁, m₂, m₃ gleich 0 sind, n₁, n₂, n₃ gleich 0 oder 1 sind und n₁+n₂+n₃ zusammen 1 bis 3 ausmachen, und dass die Sulfonat-Gruppen in meta-Stellung stehen.

4. Verfahren gemäß einem oder mehreren der Ansprüche 1 bis 3, **dadurch gekennzeichnet, dass** als Übergangsmetallverbindungen der Gruppe VIII des Periodensystems der Elemente Verbindungen von Rhodium, Kobalt, Iridium, Nickel, Palladium, Platin, Eisen oder Ruthenium verwendet werden.

5. Verfahren gemäß einem oder mehreren der Ansprüche 1 bis 4, **dadurch gekennzeichnet, dass** als Übergangsmetallverbindungen der Gruppe VIII des Periodensystems der Elemente Verbindungen von Rhodium eingesetzt werden.

6. Verfahren gemäß einem oder mehreren der Ansprüche 1 bis 5, **dadurch gekennzeichnet, dass** die Reaktionstemperatur 100 bis 150°C, bevorzugt 110 bis 140°C, und der Druck 1 bis 6 MPa, bevorzugt 1,5 bis 5 MPa, beträgt.

7. Verfahren gemäß einem oder mehreren der Ansprüche 1 bis 6, **dadurch gekennzeichnet, dass** die Rhodium-Konzentration 20 bis 1000 Gew.ppm, vorzugsweise 50 bis 800 Gew.-ppm und insbesondere 100 bis 600 Gew.-ppm, jeweils bezogen auf die wässrige Katalysatorlösung, beträgt.

8. Verfahren zur Herstellung gemäß einem oder mehreren der Ansprüche 1 bis 7, **dadurch gekennzeichnet, dass** je mol Rhodium 10 bis 300 mol, insbesondere 50 bis 150 mol, Phosphor in Form der wasserlöslichen organischen Phosphorverbindung verwendet werden.
